# EUROPEAN PATENT APPLICATION

(11) **EP 4 670 950 A1**
(43) Date of publication of application: **31.12.2025**
(21) Application number: 25181926.4
(22) Date of filing: 11.06.2025
(51) Int. Cl.: B29C 49/00, B29C 49/48, B29C 49/36, A61M 5/28, B29C 49/58, B29C 49/64, B29C 51/10, B29C 49/02, B29C 49/78, B29K 105/00, B29L 31/00

(54) **DEVICE FOR MAKING A BYPASS CHANNEL IN SYRINGE PREFORMS AND METHOD FOR MAKING A BYPASS CHANNEL IN SYRINGE PREFORMS**

(30) Priority: 25.06.2024 IT 202400014527
(71) Applicant: STEVANATO GROUP S.P.A., 35017 Piombino Dese (PD) (IT)
(72) Inventor: ZIERO, Paolo, 35017 Piombino Dese (Padova) (IT); PERIN, Fabio, I31050 Vedelago (Treviso) (IT)
(74) Representative: Porta & Consulenti Associati S.p.A.

(57) **Abstract**

A device (10) for making a bypass channel (101) in syringes comprises a gripping equipment (11) having a gripping axis (X) and comprising gripping elements (12); at least a heating station (23) comprising a heater (24) configured to emit a heating flow directed radially towards said gripping axis (X); at least one blow moulding station (25) comprising a mould (26) having a portion counter-shaped to a bypass channel (101); a blower (33) configured to deliver pressurized fluid inside the syringe preform (100), when the mould (26) is in a closed condition.

## Description

The present invention concerns a device for making a bypass channel in syringe preforms and a method for making a bypass channel in syringe preforms.

The purpose of making a bypass channel in the syringes is to make available so-called "double chamber" syringes that can be used to administer preparations composed of two components that must be mixed together before administration. These two components may be two liquid components, or a liquid component and a solid component typically in granular or powder form.

The bypass channel allows to mix the components without transferring them to other containers and to administer them directly with the syringe containing the two components.

A syringe provided with a bypass channel has a containment chamber, generally cylindrical, separated into a first chamber and a second chamber by a separation element made of elastomeric material placed in the second chamber. In the area of the first chamber there is the bypass channel which is made by one or more bulges in the side wall of the first chamber. In the second chamber there is arranged a piston that closes the second chamber towards the outside. When a substance placed in the second chamber is moved towards the first chamber by operating the piston, the separation element is also moved until it reaches the portion affected by the bypass channel. The separation element, when it reaches the portion affected by the bypass channel, is no longer able to divide in a fluid-tight manner the first and second chambers and the component placed in the second chamber is able to pass into the first chamber through the bypass channel mixing with the component placed in the first chamber. By continuing to operate the piston, the latter comes into contact with the separation element sending all the component placed in the second chamber into the first chamber. By further continuing to operate the piston, the piston and the separation element move together towards the outlet end of the syringe, passing the bypass channel and allowing the dispensing and administration of the preparation in which the first and the second component have been mixed with each other.

Document EP 3556538 B1 discloses a device and method for making a bypass channel in a syringe. In accordance with this document, a syringe preform is inserted into a mould and a portion of the cylindrical surface of the preform is heated with a heat source. A punch is inserted into the syringe preform which punch, in combination with the mould, plastically deforms the heated portion of the cylindrical surface of the preform making a bypass channel.

The Applicant has noted that, if the syringes in which to make the bypass channel have a particularly small diameter, it may be difficult to insert the punch into the syringe preform to deform the cylindrical surface of the syringe preform and make the bypass channel.

The Applicant has therefore felt the need to make available a device for making a bypass channel in syringe preforms and a method for making a bypass channel in syringe preforms that can be effectively used regardless of the size of the syringes, particularly even with small diameter syringes.

The Applicant has found that by retaining the syringe preform at one end it is possible to expose a syringe preform portion, not retained and on which the bypass channel must be obtained, to a heat source up to a softening temperature of the syringe preform portion. By blowing a pressurized fluid into the syringe preform, the portion placed at the softening temperature tends to deform and swell. By retaining the syringe preform portion in a mould in which a bypass channel mould has been obtained, the deformation of the portion placed at the softening temperature assumes the shape of the bypass channel. In this way, it is not necessary to introduce any solid body inside the syringe preform and it is therefore possible to make bypass channels in syringe preforms of any size.

The present invention therefore concerns, in a first aspect thereof, a device for making a bypass channel in syringe preforms comprising:
a gripping equipment having a gripping axis and comprising gripping elements movable between a gripping condition in which the gripping elements are radially approached to said gripping axis and a release condition in which the gripping elements are radially distanced from said gripping axis, wherein said gripping elements act at a first axial zone of said gripping axis and wherein said gripping elements are configured to grasp and retain a syringe preform when the gripping elements are in the gripping condition;
at least one heating station comprising a heater configured to emit a heating flow directed radially towards said gripping axis at a second axial zone that is distinct and axially separated from said first axial zone of the gripping axis;
at least one blow moulding station comprising:
   a mould having a portion counter-shaped to a bypass channel, wherein the mould is movable between an open condition and a closed condition, wherein in the closed condition the mould is configured to surround the syringe preform and is closed around said gripping axis at a third axial zone of the gripping axis comprising said second axial zone of the gripping axis;
   a blower configured to deliver pressurized fluid inside said syringe preform, wherein said blower is switchable between an active condition in which it delivers a direct pressurized fluid along said gripping axis when the mould is in the closed condition and an inactive condition in which it does not deliver pressurized fluid when the mould is in the open condition.

The Applicant has further noted that the proposed solution is particularly advantageous when the syringe preforms are made of glass or similar material.

The Applicant has in fact verified that when a portion of the glass syringe preform is heated, tensions internal to the syringe preform are generated due to the temperature difference between the heated portion and the remaining portions of the syringe preform. The Applicant has noted that when the syringe preform is retained at several portions that are axially distal between them during heating of the portion on which the bypass channel is to be formed, such internal stresses tend to crack the syringe preform and thus render the syringe preform unusable.

The Applicant has found that by arranging the gripping elements of the gripping equipment in such a way that they act at a first axial zone of the syringe preform and by arranging a heater in such a way that it emits a heating flow at a second axial zone that is distinct and axially separated from said first axial zone, the possibility of formation of cracks and breakages of the syringe preform is drastically reduced.

Without wishing to be bound by any particular scientific theory, the Applicant considers that the tensions internal to the syringe preform that are generated during heating can be discharged at the free and unretained end of the syringe preform, since the latter is retained at the first axial zone during heating.

The present invention concerns, in a second aspect thereof, a method for making a bypass channel in syringe preforms comprising:
retaining, with a gripping equipment, a syringe preform through an end of the syringe preform;
heating with a heating flow a portion of the syringe preform while being retained by the gripping equipment;
arranging the syringe preform in a mould having a portion counter-shaped to a bypass channel by orienting the syringe preform with the heated portion at the bypass channel;
blowing pressurized fluid inside the syringe preform while it is placed in said mould.

In the present description and in the appended claims, with the term "axial, axially" when referring to an axis, it is meant a direction or an orientation aligned, parallel or coinciding with that axis.

In the present description and in the appended claims, with the term "radial, radially" when referring to an axis, it is meant a direction or an orientation substantially perpendicular to such axis and contained in a plane perpendicular to such axis.

In the present description and in the appended claims, with the term "circumferential, circumferentially" when referring to an axis, it is meant a direction or an orientation substantially contained in a plane perpendicular to said axis and which develops around said axis.

Preferred features of the present invention are recited below, each of these features may be provided individually or in combination with the others.

Preferably, the first axial zone is placed at a flange of said syringe preform.

Preferably, the gripping axis coincides with a development axis of the syringe preform when the syringe preform is retained by the gripping equipment.

Preferably, said first axial zone substantially coincides with a portion close to a first end of the syringe preform.

Preferably, said portion close to a first end of the syringe preform comprises a flange of the syringe preform.

Preferably, said second axial zone of the gripping axis is substantially coincident with a portion of the syringe preform placed in proximity to a cone of the syringe preform.

Preferably, the gripping elements of the gripping equipment act only at the first axial zone of the gripping axis to retain the syringe preform only at a portion close to a first end of the syringe preform.

Preferably, said mould comprises a first half-part and a second half-part having a substantially cylindrical mould inner surface.

Preferably, the second half-part comprises an open cavity on said inner mould surface defining said bypass channel.

When the mould is closed around the gripping axis and thus around the syringe preform, the heated syringe preform portion is placed at the cavity of the second half-part of the mould.

Preferably, the extension in the axial direction of the third axial zone is comprised between 100% and 120% of the extension in the axial direction of the second axial zone.

In this way, the mould is sized to surround the syringe preform substantially only in the zone where the bypass channel is to be made, allowing the use of a mould of small size and therefore economical to make.

Preferably, said blower is selectively associated in fluid connection with a blowing nozzle placed along said gripping axis and between said gripping elements of the gripping equipment.

In this way, the blowing nozzle is positioned at an end of the syringe preform and faces inside the syringe preform.

By operating the blower, pressurized fluid enters the syringe preform and plastically deforms the portion of the heated preform by making it adhere to the cavity of the second half-part of the mould.

Preferably, a carousel rotating around a carousel axis and a plurality of said gripping equipment mounted on said carousel radially around said carousel axis are provided.

Preferably, the gripping axes of each gripping device are parallel to the carousel axis.

The Applicant has found that by mounting a plurality of gripping equipment on the rotating carousel, the syringe preforms can be moved, each retained by a respective gripping equipment, together with the rotating carousel.

In this way it is for example possible to arrange the heating station and the blow moulding station along a path followed by the gripping equipment transported by the rotating carousel and to implement the heating of a syringe preform while another already heated syringe preform is placed in the moulding station.

The Applicant has verified that in this way it is possible to increase, in the unit of time, the number of bypass channels formed on respective syringe preforms.

Preferably, a plurality of heating stations placed radially around said carousel axis and not rotatable with said carousel are provided.

Preferably, the heating stations follow each other circumferentially around the carousel axis.

Preferably, the heating stations are arranged along a path followed by the gripping equipment during the rotation of the rotating carousel.

Preferably, the number of gripping equipment is greater than the number of heating stations.

Preferably, a number D of gripping equipment of said plurality of gripping equipment and a number R of heating stations being in a relationship where D is greater than or equal to R+2.

Preferably, the heating flows of the heaters of two adjacent and circumferentially successive heating stations have different temperatures.

More preferably, the temperatures of the heating flows of the heaters of two adjacent and circumferentially successive heating stations are growing in the direction of the path followed by the gripping equipment during the rotation of the rotating carousel.

The Applicant has found that this allows to gradually heat the portion of the syringe preform on which the bypass channel is to be made, reducing the possibility of breakage of the syringe preforms due to too high thermal shocks.

Preferably, the gripping elements of each gripping equipment are rotatable about their respective gripping axes during the rotation of the carousel to expose a same portion of the syringe preform to the heaters of the heating stations.

In this way it is possible to heat only the portion of the syringe preform on which the bypass channel is to be made without having to orient the heaters of the heating stations from time to time.

Preferably, two heaters of two adjacent and circumferentially successive heating stations are angularly spaced apart by a first angle.

Preferably, the gripping elements of a gripping equipment rotate about said gripping axis by an angle equal to said first angle during a rotation of the carousel moving said gripping equipment between said two heating stations.

In this way, the syringe preform is transported by the gripping equipment always exposing the same portion to the heaters of the heating stations.

In the case where the preforms are made of glass, preferably the temperatures of the heating flows of the heaters of the heating stations grow along the path followed by the gripping equipment between about 150 °C and about 900 °C, more preferably between about 200 °C and about 800 °C.

Preferably, there is provided a pick-up station reached by each gripping equipment during the rotation of the carousel around the carousel axis.

When a gripping equipment reaches the pick-up station, a syringe preform on which the bypass channel is to be created is associated with the gripping equipment.

Preferably, the gripping elements of each gripping equipment move from the release condition to the gripping condition when they reach said pick-up station in such a way as to pick up a syringe preform.

Preferably, the temperature of the heating flow of the heater of the heating station closest to the pick-up station is lower than the temperature of the heating flow of the heater of the heating station most distal from the pick-up station.

Preferably, a syringe preform is moved at the pick-up station to be able to insert it between the gripping elements of the gripping equipment.

Preferably, said pick-up station comprises a group of actuators configured to insert a syringe preform into the gripping equipment placed at the pick-up station.

Preferably, the group of actuators is configured to lift and subsequently translate a syringe preform when the latter is in the pick-up station.

Preferably, the group of actuators comprises a first actuator movable along a direction perpendicular to the gripping axis of the gripping equipment and configured to lift a syringe preform.

Preferably, the group of actuators comprises a second actuator movable along the gripping axis of the gripping equipment and configured to translate a syringe preform to insert the syringe preform between the gripping elements.

Preferably, the second actuator is activated after the first actuator.

The Applicant has perceived that in order to increase the production rate of bypass channels in the syringe preforms it is advantageous to continuously feed the syringe preforms on which the bypass channel is to be made to the pick-up station.

For this purpose, a conveyor is preferably provided which reaches said pick-up station and which comprises a plurality of transport seats configured to transport syringe preforms.

Preferably, each transport seat comprises a pair of supports spaced apart from one another along a lying axis.

The lying axis coincides with the development axis of the syringe preform when it is transported to a transport seat.

Preferably, the pair of supports acts on a first portion and a second portion of the lying axis axially spaced apart from one another.

Preferably, the first portion of the lying axis corresponds to a portion of the syringe preform close to the flange of the syringe preform.

Preferably, the second portion of the lying axis corresponds to a portion of the syringe preform close to the cone of the syringe preform.

The Applicant has noted that, in the case of syringe preforms having a flange with circular portions and straight portions, the syringe preforms do not exhibit a perfect cylindrical symmetry. In these cases, the position of the bypass channel cannot be randomly chosen on the cylindrical surface of the syringe preform. Indeed, the position of the bypass channel on the cylindrical surface of the syringe preform may require a particular and predetermined position with respect to the straight portion of the flange.

The Applicant has noted that it is therefore necessary to correctly orient the syringe preform by rotating it around its development axis in order to correctly orient the syringe preform with respect to the mould bearing the bypass channel, so that the bypass channel is made in the correct position of the syringe preform.

The Applicant has verified that this operation may require a complex system for monitoring the orientation of the position of the syringe preform and a complex system for orientation of the syringe preform.

The Applicant has perceived that if it were possible to ensure that the syringe preforms are transported to the pick-up station with a predetermined orientation and that the syringe preforms are transferred to the pick-up station from the conveyor to the gripping equipment without changing this predetermined orientation, it would be possible to avoid having to create complex and expensive orientation and monitoring systems for the syringe preform.

The Applicant has found that by providing the conveyor and/or the transport seats with a guide element configured to receive in abutment the flange of the syringe preforms, said guide element could be used as a rest element for the straight part of the flange. In this way, the syringe preforms would all be oriented in the same way on the conveyor, i.e. with the straight portion of the flange in abutment on the guide element, making it easy to determine the position on which to make the bypass channel.

Thus, preferably, the conveyor comprises guide elements configured to receive in abutment flanges of the syringe preforms.

Preferably, said guide elements are developed along the entire conveyor so as to make a single substantially flat rest shelf.

Preferably, said shelf is configured to receive in abutment a straight portion of the flange of the syringe preform.

Preferably, the pair of supports of each transport seat is subsequent to said guide element along the lying axis such that the guide element is arranged at one end of the syringe preform.

Preferably, in said pick-up station the laying axis of a transport seat is parallel to the gripping axis of a gripping equipment.

Preferably, said group of actuators does not rotate the syringe preform about a development axis thereof during lifting and translation of the syringe preform.

Preferably, an unloading station is provided which is reached by each gripping equipment during the rotation of the carousel around the carousel axis.

The unloading station is configured such that each syringe preform on which the bypass channel has been made is picked up by the respective gripping equipment.

Preferably, the gripping elements of each gripping equipment move from the gripping condition to the release condition when they reach said unloading station in such a way as to release a syringe preform.

Preferably, the unloading station is substantially identical to the pick-up station.

Preferably, the unloading station comprises a further group of actuators configured to extract a syringe preform from the gripping equipment placed at the unloading station.

Preferably, the further group of actuators is configured to translate and subsequently lower a syringe preform when the latter is in the unloading station.

Preferably, the further group of actuators comprises a first actuator movable along the gripping axis of the gripping equipment and configured to translate a syringe preform axially away from the gripping elements.

Preferably, the further group of actuators comprises a second actuator movable along a direction perpendicular to the gripping axis of the gripping equipment and configured to lower a syringe preform.

Preferably, the conveyor reaches the unloading station to receive syringe preforms from the unloading station.

Preferably, a control station configured to identify at least geometric characteristics of the bypass formed on the syringe preform is provided.

In this way it is possible to quickly detect if any anomaly has occurred in the device such as to prevent the correct realization of the bypass channels and immediately stop the device avoiding producing a high amount of bypass channels and therefore avoiding having to discard entire production batches.

Preferably, the control station is placed along the conveyor subsequently to the unloading station.

The control station may comprise at least one optical inspection device.

Preferably, a reject station configured to reject a syringe preform whose bypass does not have geometric characteristics conforming to a predetermined geometric pattern is provided.

This prevents syringe preforms with incorrectly made bypass channels from being incorrectly inserted into production batches intended for sale.

Preferably, the control station is placed along the conveyor subsequently to the control station.

Further characteristics and advantages of the present invention will become clearer from the following detailed description of the preferred embodiments thereof, with reference to the appended drawings and provided by way of indicative and non-limiting example. In such drawings:
- figure 1 represents a schematic front view of a device for making a bypass channel in syringes;
- figure 2 is a is a schematic perspective view of a gripping equipment of the device of figure 1;
- figure 3 is a schematic perspective view of some details of the gripping equipment of figure 2;
- figure 4 is a schematic perspective view of a blow moulding station of the device of figure 1;
- figure 5 is a schematic perspective view of some details of the blow moulding station of figure 4;
- figure 6 is a schematic perspective view of a conveyor of the device of figure 1;
- figure 7 is a schematic side view of a pick-up station of the device of figure 1;
- figure 8 is a schematic side view of an unloading station of the device of figure 1; and
- figure 9 is a schematic perspective view of a syringe preform with bypass channel.

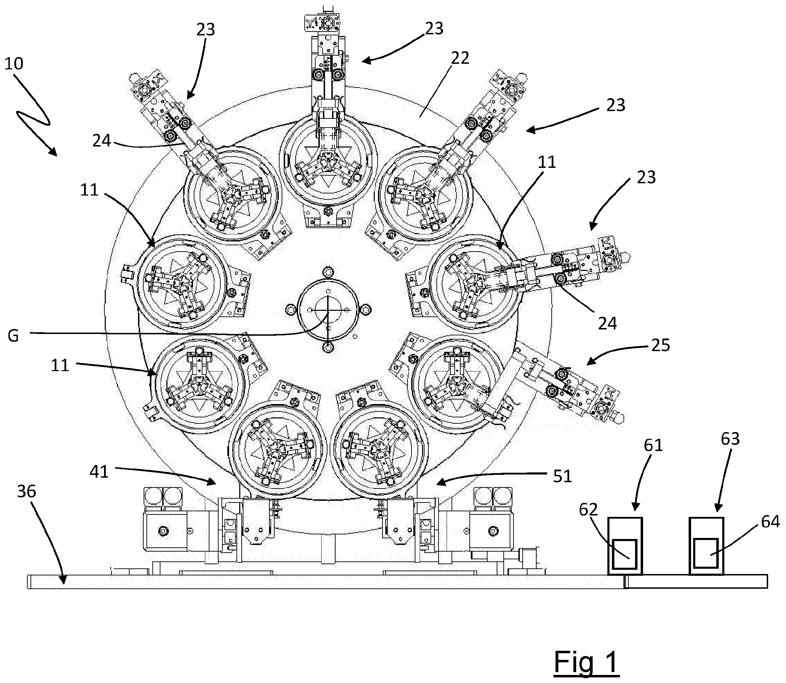

In figure 1, 10 denotes a device for making a bypass channel in syringes in accordance with the present invention.

The device 10 is configured to pick up syringe preforms 100 and to make a bypass channel 101 on the syringe preforms 100.

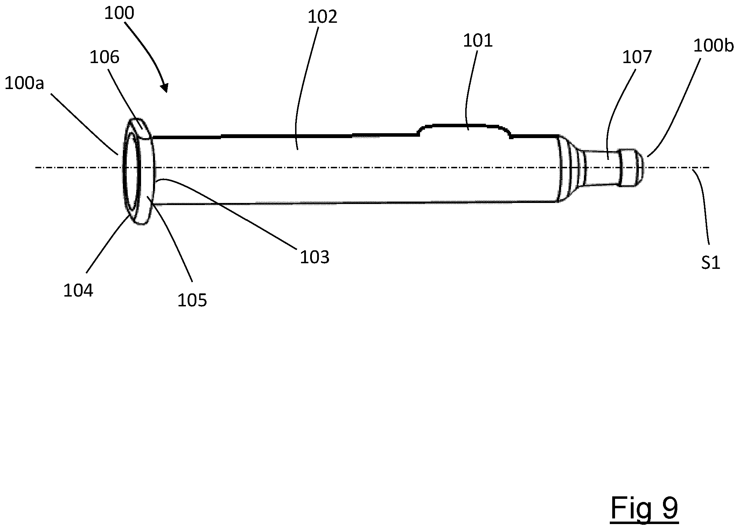

Figure 9 schematically illustrates a syringe preform 100 with a bypass channel 101. The syringe preform 100 is generally cylindrical in shape and has a main development axis S1. The syringe preform 100 comprises a generally cylindrical side wall 102. At a first end 100a, the syringe preform 100 comprises a flange 103. The flange 103 comprises a collar 104 that develops radially away from the side surface 102. In the embodiment of syringe preform 100 illustrated in figure 9, the collar 104 comprises two opposite curved portions 105 joined by two straight portions 106 which are also opposite one another. At a second end 100b opposite the first end 100a, the syringe preform comprises a cone 107 which, in use, defines an outlet opening for a medicament to be dispensed through the syringe. Typically, an injection needle (not illustrated) may be associated to the cone 107. In proximity to the cone 107 there is placed the bypass channel 101. The bypass channel 101 is made by a radial protuberance of the side surface 102.

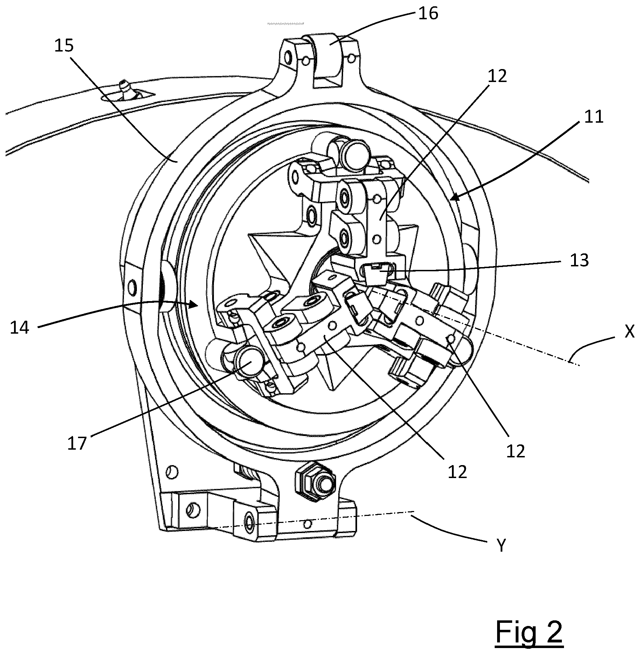

The device 10 comprises at least one gripping equipment 11 (better illustrated in figure 2) configured to retain the syringe preform 100 during the operations of forming the bypass channel 101. The gripping equipment 11 is configured to retain the syringe preform 100 only at a portion of the latter. In particular, the gripping equipment 11 is configured to retain the syringe preform 100 at the first end 100a of the syringe preform 100 where the flange 103 is provided.

In this regard, the gripping equipment 11 comprises a substantially straight gripping axis X. The gripping axis X coincides with the development axis S1 of the syringe preform 100 when the latter is retained by the gripping equipment 11. The gripping equipment 11 comprises a plurality of gripping elements 12 which are movable between a gripping condition and a release condition. In the gripping condition the gripping elements 12 are approached to the gripping axis X and in the release condition the gripping elements 12 are distanced from the gripping axis X. In the preferred embodiment of the invention, three gripping elements 12 are provided arranged circumferentially around the gripping axis X. The gripping elements 12 are equidistantly spaced from one another by an angle of 120°. Each gripping element 12 comprises a gripping pad 13 configured to contact the side wall 102 of the syringe preform 100. Each gripping pad 13 is counter-shaped to a portion of the side surface 102 of the syringe preform 100. Each gripping pad 13 is for example made of graphite.

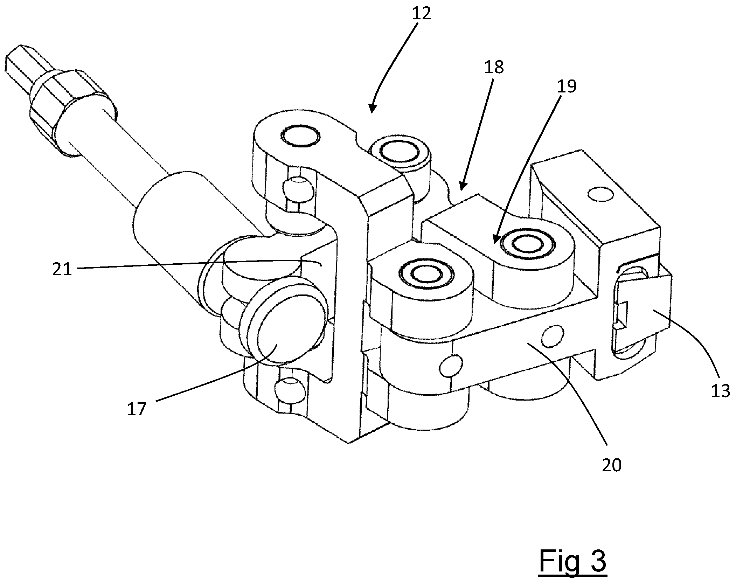

In order to move the gripping elements 12 between the gripping condition and the release condition, the gripping equipment 11 comprises a movement mechanism 14. The movement mechanism 14 comprises a collar 15 that circumferentially surrounds the gripping elements 12. The collar 15 is hinged so that it can rotate about a hinge axis Y substantially perpendicular to the gripping axis X. In a position radially opposite to the hinge axis Y, the collar 15 comprises a feeler 16. The movement mechanism 14 further comprises a slider 17 for each gripping element 12. The sliders 17 are movable in a direction parallel to the gripping axis X. When the feeler 16 is pressed in a direction parallel to the gripping axis X, the collar 15 rotates about the hinge axis Y and causes the translation of the sliders 17. A return spring holds the feeler 16 in a normally unpressed condition. Each slider is active on a respective kinematics 18 of the respective gripping element 12. This kinematics 18 (better illustrated in figure 3) comprises a four-bar linkage 19 on whose first rod 20 the slider 17 acts and to which second rod 21 the gripping pad 13 is constrained. The translation of the slider 17 moves the four-bar linkage 19 by radially approaching or radially distancing (depending on the translation direction of the slider 17) the gripping pad 13 to the gripping axis X.

As illustrated in figure 1, the device 10 comprises a plurality of gripping equipment 11 each of which mounted on a carousel 22 rotating about a carousel axis G parallel to the gripping axes X of each gripping equipment 11. The gripping axes X of the gripping equipment 11 are parallel to each other. When the carousel 22 rotates around the carousel axis G, the gripping equipment 11 move with the carousel 22.

The gripping equipment 11 follow a substantially circular path (directed clockwise for the viewer of figure 1) during the rotation of the carousel 22. The gripping equipment 11 are mounted around the carousel axis G and are radially equidistant from the carousel axis G. The carousel 22 rotates around the carousel axis G with a step-by-step rotation, i.e. it moves with continuous rotations that are spaced out from one another by dwell times in which the carousel 22 does not rotate.

Circumferentially around the carousel 22, the device 10 comprises a plurality of heating stations 23. Four heating stations 23 are depicted in figure 1, however embodiments may be provided in which the heating stations are 1 to 5 in number, for example 3. The heating stations 23 are not movable with the carousel 22 but are fixed, possibly being able to move in a radial direction towards the carousel axis G. The heating stations 23 follow one another along the path followed by the gripping equipment 11 during the rotation of the carousel 22.

Each heating station 23 comprises a heater 24 configured to emit a heating flow along a direction generally aligned to a radial direction directed towards the carousel axis G. Each heater 24 has the function of heating the portion of side surface 102 of the syringe preform 100 on which the bypass channel 101 is to be made. For this purpose, in the preferred embodiment of the invention which provides glass syringe preforms, each heater 24 is a burner that delivers a flame which defines said heating flow. The flame temperature of the heaters 24 is growing along the path followed by the gripping equipment 11, in such a way as to progressively heat up to a softening temperature the portion of the side surface 102 of the syringe preform 100 on which the bypass channel 101 is to be made. By way of example, the flame temperature of the heaters 24 grows from about 200°C at the first heater encountered by the gripping equipment 11 up to reaching about 800°C at the last heater 24 encountered by the gripping equipment 11 during the rotation of the carousel 22.

To ensure that in the heating stations 23 only the portion of the side surface 102 of the syringe preform 100 on which the bypass channel 101 is to be made is heated, the gripping equipment 11 are rotatable about the respective gripping axes X. In particular, the gripping elements 12 of each gripping equipment 11 are rotatable about the respective gripping axes X during the rotation of the carousel 22 carrying the gripping equipment 11 between one heating station 23 and the next. The amount of the rotation of the gripping elements 12 is chosen such that always the same portion of the side surface 102 of the syringe preform 100 is exposed to the flame of the heaters 24. By way of example, the gripping elements 12 of a gripping equipment 11 rotate around the gripping axis X by an angle equal to the angle separating two adjacent and circumferentially successive heating stations. The rotation of the gripping elements 12 takes place only during the rotation of the carousel 22. When the gripping equipment 12 reach a respective heating station 23, the rotation of the gripping equipment 12 is interrupted.

Circumferentially around the carousel 22, the device 10 further comprises a blow moulding station 25.

The blow moulding station 25 is placed subsequently to the heating stations 23 along the path followed by the gripping equipment 11 during the rotation of the carousel 22. The blow moulding station 25 has the function of plastically deforming the portion of the side surface 102 of the syringe preform 100 to make the bypass channel 101.

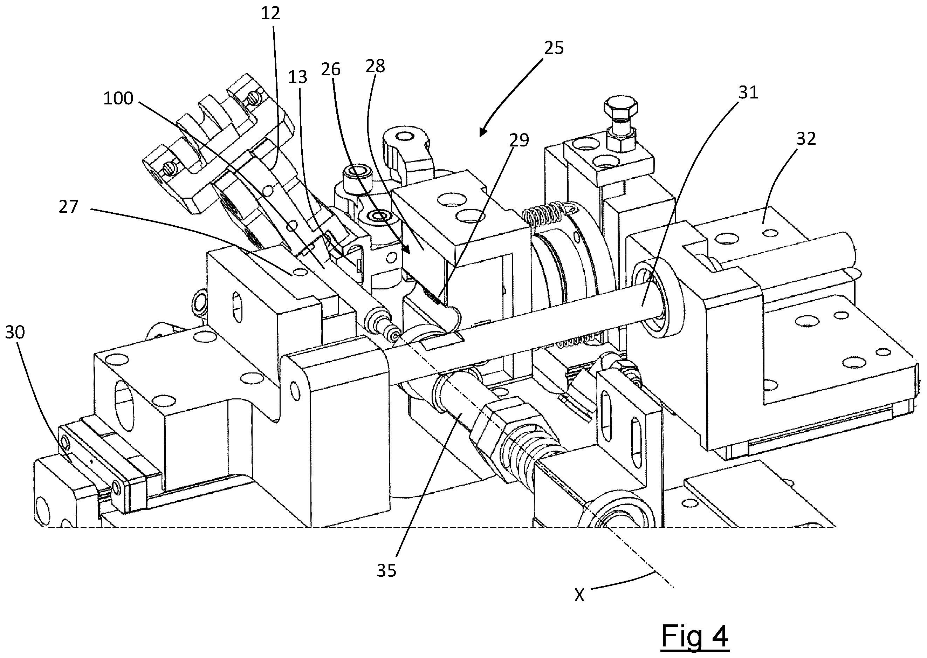

In this regard, the blow moulding station 25 comprises a mould 26 that closes around the side surface 102 of the syringe preform 100. The mould 26 comprises a first half-part 27 and a second half-part 28 (figure 4). Both the first half-part 27 and the second half-part 28 have a substantially semi-cylindrical inner surface and in any case counter-shaped to the side surface 102 of the syringe preform 100. The second half-part 28 comprises a cavity 29 open on the inner surface that is counter-shaped to the bypass channel 101 to be made. When the mould 26 is closed on the syringe preform 100, the cavity 29 is directly facing the heated portion of the side surface 102 of the syringe preform 100.

The first half-part 27 and the second half-part 28 are movable towards the gripping axis X of a gripping equipment 12 when the latter reaches the blow moulding station 25. For this purpose, the first half-part 27 is mounted on a first track guide 30 in order to be able to translate closer to the gripping axis X of the gripping equipment 12 and therefore to the syringe preform 100. An electric or pneumatic actuator (not illustrated) moves through a movement rod 31 the first half-part 27 along the first track guide 30. Similarly, the second half-part 28 is mounted on a second track guide 32 in order to be able to translate closer to the gripping axis X of the gripping equipment 12 and therefore to the syringe preform 100. An electric or pneumatic actuator (not illustrated) moves the second half-part 28 along the second track guide 32.

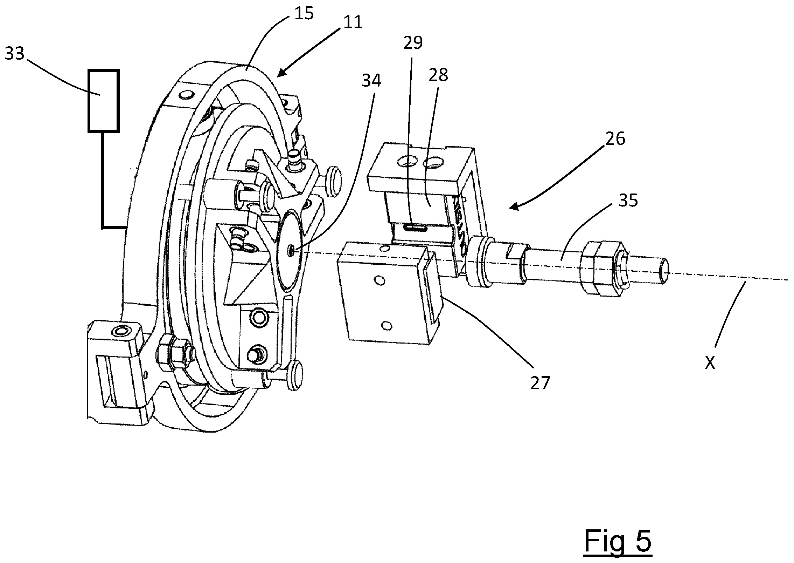

In the blow moulding station 25 a blower 33 is also active (only schematized in figure 5) configured to deliver pressurized fluid inside the syringe preform 100 on which the mould 26 has been closed. The blower 33 is placed in fluid connection with a blowing nozzle 34 placed on the gripping equipment 11. The blower 33 is placed in fluid connection with the blowing nozzle 34 when the gripping equipment 11 is in the blow moulding station 25. As schematically illustrated in figure 5, the blowing nozzle 34 is placed along the gripping axis X of the gripping equipment 11, so that pressurized fluid can be blown into the syringe preform 100. The blower 33 is switchable between an active condition in which it delivers the pressurized fluid when the mould 26 is in the closed condition and an inactive condition in which it does not deliver pressurized fluid when the mould 26 is in the open condition. The blow moulding station 25 further comprises a shutter 35 configured to close the opening of the syringe preform 100 at the cone 107. The shutter 35 develops along the gripping axis X when the gripping equipment 11 is in the blow moulding station 25 and on the opposite side with respect to the blowing nozzle 34. The pressurized fluid delivered by the blower 33 is preferably compressed air or a compressed inert gas (e.g. nitrogen). The blowing pressure exiting from the blowing nozzle 34 is comprised between about 0.5 bar and about 5 bar.

As schematically illustrated in figure 1, the device 10 comprises a conveyor 36. The conveyor 36 is configured to feed in succession syringe preforms 100 towards the gripping equipment 11. Preferably, the conveyor 36 is a ring-closed conveyor belt on respective pulleys.

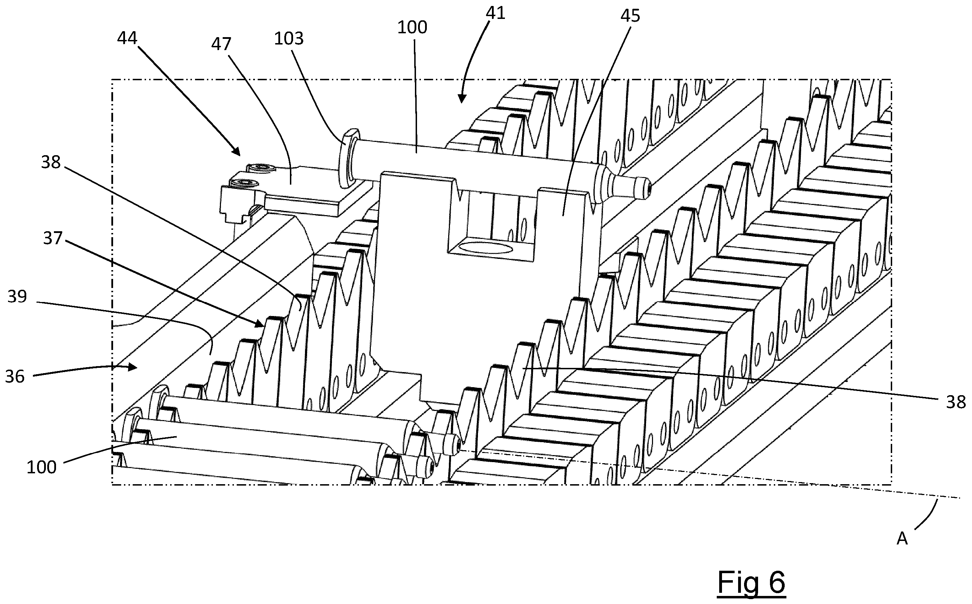

As best illustrated in figure 6, the conveyor 36 comprises a plurality of transport seats 37 configured to transport syringe preforms 100. Each transport seat 37 comprises a pair of supports 38 spaced apart from one another along a lying axis A of the syringe preform 100 on the conveyor 36. The supports are substantially "V" shaped, as depicted in figure 6 to contact portions of the syringe preform comprised between the flange 103 and the cone 107.

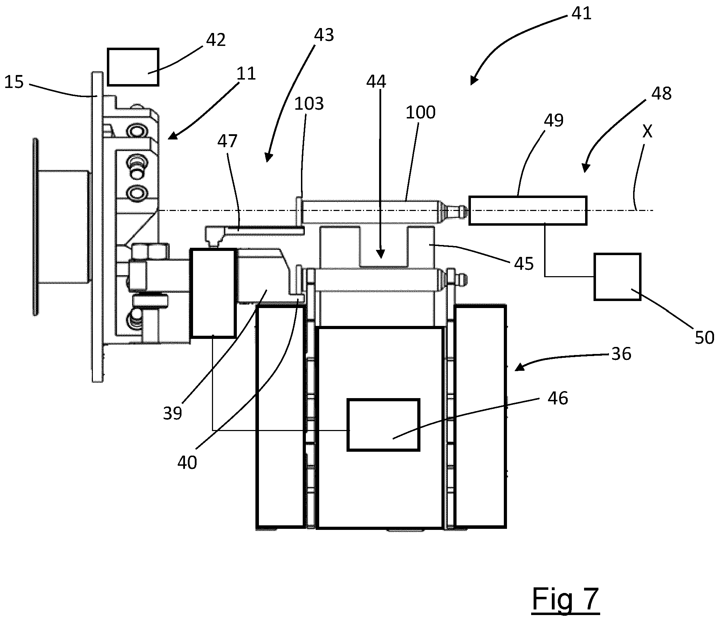

As schematically illustrated in figure 7, to transport the syringe preforms 100 according to a predefined and spatial orientation equal for all syringe preforms 100, the conveyor 36 comprises guide elements 39 configured to receive in abutment straight portions 106 of the flanges 103 of the syringe preforms 100. The guide elements 39 develop along the entire conveyor 36 and define a single substantially flat rest shelf 40.

As schematically illustrated in figure 6, the supports 38 of the guide seats 37 are arranged subsequently to the guide element 39 along the lying axis A, in such a way that the guide element 39 contacts and supports the flange 103 of the syringe preform 100 maintaining the syringe preform 100 according to a predefined spatial orientation by virtue of the straight portion 106 of the flange 103 in abutment on the rest shelf 40.

As schematically illustrated in figure 1, the device 10 comprises a pick-up station 41. The pick-up station 41 is configured to give in succession to the gripping equipment 11 a respective syringe preform 100.

The pick-up station 41 is reached by the gripping equipment 11 during the rotation of the carousel 22. When a gripping equipment 11 reaches the pick-up station, the gripping elements 12 are switched into the gripping condition ready to receive a syringe preform 100.

For this purpose, the pick-up station 41 comprises an abutment element 42 (only schematized in figure 7) which is intercepted by the feeler 16 of the gripping equipment 11 and which presses the feeler 16 in a direction parallel to the gripping axis X. The feeler 16 causes the rotation of the collar 15 and, through the sliders 17 and the four-bar linkage 19, translates the gripping pads 13 in a radial direction towards the gripping axis X, grasping and retaining a syringe preform 100.

Before or during the actuation of the feeler 16, the pick-up station 41 places a syringe preform 100 along the gripping axis X with the flange 103 in proximity to the gripping elements 12 of the gripping equipment 11.

For this purpose, the pick-up station 41 comprises a group of actuators 43 configured to insert a syringe preform 100 into the gripping equipment 11 placed at the pick-up station 41.

The group of actuators 43 comprises a first actuator 44 which is movable along a direction perpendicular to the gripping axis X and which lifts a syringe preform 100 transported by the conveyor 36. The first actuator 44 comprises a pair of lifting supports 45 placed axially inside the pair of supports 38 of the transport seats 37 (as schematically illustrated in figure 6). An electric actuator 46 (only schematized in figure 7) of the first actuator 44 is active on the pair of lifting supports 45 to lift the pair of lifting supports 45 and thus to pick up the syringe preform 100 from the transport seat 37 and to bring it in elevation above the conveyor 36. The first actuator 44 further comprises a shelf 47 which is movable between a lowered condition and a raised condition. In the lowered condition, the shelf 47 lies aligned with the guide element 39 and supports in abutment the straight portion 106 of the flange 103 of the syringe preform 100 that has reached the pick-up station 41. The electric actuator 46 (or another electric actuator) acts on the shelf 47 to translate it in a direction perpendicular to the gripping axis X and switch the shelf 47 into the raised condition. The shelf 47 is lifted simultaneously with the pair of lifting supports 45 so that the straight portion 106 of the flange 103 of the syringe preform 100 continues to rest, so that the syringe preform 100 maintains the predetermined spatial orientation.

The group of actuators 43 further comprises a second actuator 48 movable along the gripping axis X of the gripping equipment 11. The second actuator 48 has the function of pushing the syringe preform 100 towards the gripping elements 12 of the gripping equipment 11. The second actuator 48 comprises a pusher 49 driven by a respective electric actuator 50 (depicted schematically in figure 7). The second actuator 48 intervenes after the first actuator 44 has lifted the syringe preform 100 to align it with the gripping axis X.

As schematically illustrated in figure 1, the device 10 further comprises an unloading station 51. The unloading station 51 is configured to pick up from the gripping equipment 11 a respective syringe preform 100 on which the bypass channel 101 has been made.

The unloading station 51 is reached by the gripping equipment 11 during the rotation of the carousel 22. When a gripping equipment 11 reaches the unloading station 51, the gripping elements 12 are switched into the release condition in order to be able to extract the syringe preform 100 from the gripping equipment 11.

The unloading station 51 is substantially structurally identical to the pick-up station 41.

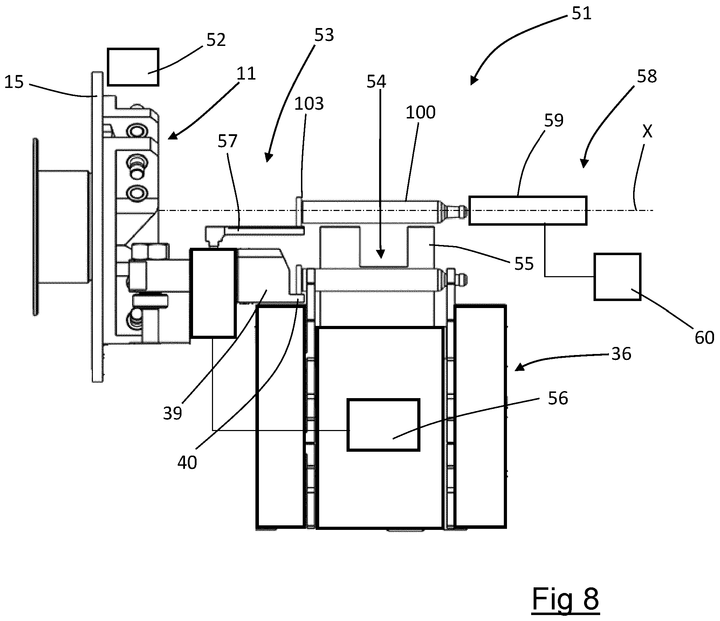

The unloading station 51 comprises an abutment element 52 (only schematized in figure 8) which is intercepted by the feeler 16 of the gripping equipment 11 and which presses the feeler 16 in a direction parallel to the gripping axis X. The feeler 16 causes the rotation of the collar 15 and, through the sliders 17 and the four-bar linkage 19, translates the gripping pads 13 in a radial direction away from the gripping axis X releasing the syringe preform 100.

The unloading station 51 comprises a further group of actuators 53 configured to extract the syringe preform 100 from the gripping equipment 11 placed at the unloading station 51.

The further group of actuators 53 comprises a first actuator 58 movable along the gripping axis X of the gripping equipment 11. The first actuator 58 has the function of pushing or pulling the syringe preform 100 axially away from the gripping elements 12 of the gripping equipment 11. The first actuator 58 comprises a pusher 59 driven by a respective electric actuator 60 (schematically represented in figure 8).

The further group of actuators 53 further comprises a second actuator 54 which is movable along a direction perpendicular to the gripping axis X and which lowers a syringe preform 100 onto the conveyor 36. The second actuator 54 comprises a pair of lifting supports 55 axially placed within a pair of supports 38 of the transport seats 37. An electric actuator 56 (only schematized in figure 8) of the second actuator 54 is active on the pair of lifting supports 55 to lower the pair of lifting supports 55 and then rest the syringe preform 100 on a transport seat 37 of the conveyor 36. The second actuator 54 further comprises a shelf 57 which is movable between a raised condition and a lowered condition. In the raised condition, the shelf 57 lies substantially aligned with the gripping axis X and supports in abutment the straight portion 106 of the flange 103 of the syringe preform 100 that has been translated by the first actuator 58 in axial distancing from the gripping equipment 11. The electric actuator 60 (or another electric actuator) acts on the shelf 57 to translate it in a direction perpendicular to the gripping axis X and switch the shelf 47 into the lowered condition. The shelf 47 is lowered simultaneously with the pair of lifting supports 55 so that the straight portion 106 of the flange 103 of the syringe preform 100 continues to rest, so that the syringe preform 100 maintains the predetermined spatial orientation when inserted into a transport seat 37 of the conveyor 36.

The second actuator 54 intervenes after the first actuator 58 has translated the syringe preform 100 along the gripping axis X.

Downstream of the unloading station 51 and along the conveyor 36 there is a control station 61 schematized in figure 1. The control station 61 is configured to identify at least geometric characteristics of the bypass channel 101 formed on the syringe preform 100.

The control station 61 comprises an optical inspection device 62. The optical inspection device 62 is preferably a camera. The camera is positioned so as to optically inspect a side profile of the bypass channel 101, and it acquires at least one image of a portion of the syringe preform 100 comprising the bypass channel 101. The acquired image is sent to a logical control unit that compares it with a reference sample image to detect any differences in geometry.

During the check, the syringe preform 100 is positioned on the conveyor 36 so that the bypass channel 101 is oriented in a predetermined manner, preferably downwards.

Downstream of the control station 61 there is provided a reject station 63 configured to reject a syringe preform 100 whose bypass channel 101 does not have geometric characteristics conforming to the reference sample image. The reject station 63 comprises a linear actuator 64 (schematized in figure 1), driven by the control logic unit, arranged laterally with respect to the conveyor 36 and operable according to a direction orthogonal to the advancement direction of the conveyor 36. When actuated, the linear actuator 64 acts abuttingly on the cone 107 of the syringe preform 100 to remove it from the conveyor 36 and push it towards a waste collection box.

In use, the syringe preforms 100 on which the bypass channels 101 are to be made are fed in a row one after the other by the conveyor 36. According to the modalities described above, the syringe preforms 100 are transported parallel to each other and with a straight portion 106 of the flange 103 in abutment on the guide element 39.

When a syringe preform 100 reaches the pick-up station 41, the syringe preform 100 is inserted into a gripping equipment 11 with the only end of the syringe preform 100 bearing the flange 103 retained by the gripping elements 12 of the gripping equipment 11. The carousel 22 is placed in rotation causing the rotation of the gripping equipment 11 about the carousel axis G.

When the gripping equipment 11 and with it the syringe preform 100 reaches a first heating station 23, the rotation of the carousel 22 is suspended and the heater 24 of the first heating station is activated by heating, for example to about 200 °C, the portion of the syringe preform 100 on which the bypass channel 101 is to be made.

At the end of the heating, the carousel 22 is again placed in rotation around the carousel axis and at the same time the gripping elements 12 are placed in rotation around the gripping axis X. When the gripping equipment 11 and with it the syringe preform 100 reaches a second heating station 23, the rotation of the carousel 22 is suspended and the heater 24 of the second heating station is activated. The rotation of the gripping elements 12 about the gripping axis X is chosen such that when the gripping equipment reaches the second heating station 23, the portion of the syringe preform 100 that is heated by the relative heater 24 is the same as the one which was heated by the first heater 24. The heater 24 of the second heating station 23 heats for example to about 500°C the portion of the syringe preform 100 on which the bypass channel 101 is to be made.

At the end of the heating, the carousel 22 is again placed in rotation around the carousel axis G and at the same time the gripping elements 12 are placed in rotation around the gripping axis X. When the gripping equipment 11 and with it the syringe preform 100 reaches a third heating station 23, the rotation of the carousel 22 is suspended and the heater 24 of the third heating station is activated. The rotation of the gripping elements 12 about the gripping axis X is chosen such that when the gripping equipment 11 reaches the third heating station 23, the portion of the syringe preform 100 that is heated by the relative heater 24 is the same as the one which was heated by the first and second heater 24. The heater 24 of the third heating station 23 heats for example to about 800 °C the portion of the syringe preform 100 on which the bypass channel 101 is to be made.

At the end of the heating, the carousel 22 is again placed in rotation about the carousel axis G until the gripping equipment 11 reaches the blow moulding station 25 where the rotation of the carousel 22 is suspended. The mould 26 is then closed on the syringe preform 100 according to the modalities described above and pressurized fluid is blown into the syringe preform 100. This causes swelling of the heated portion of side wall 102 of the syringe preform 100 which adheres to the cavity 29 of the mould 26 thereby making the bypass channel 101. When this operation has been completed, the mould 26 is opened and the carousel 22 is again placed in rotation about the carousel axis G. When the gripping equipment 11 reaches the unloading station 51, the rotation of the carousel 22 is interrupted and the syringe preform 100 bearing the bypass channel 101 is extracted from the gripping equipment 11 according to the modalities described above. The syringe preform 100 bearing the bypass channel 101 is then laid on the conveyor 36 and subsequently passes through the control station 61 and the reject station 63.

Note that each rotation of the carousel 22 brings a respective gripping equipment 11 to a corresponding station. In other words, the heating stations 23, the blow moulding station 25, the pick-up station 41 and the unloading station 51 are always occupied by a respective gripping equipment 11 and by a respective syringe preform 100 at the end of each rotation step of the carousel 22.

## Claims

1. Device (10) for making a bypass channel (101) in syringe preforms (100) comprising:
a gripping equipment (11) having a gripping axis (X) and comprising gripping elements (12) movable between a gripping condition in which the gripping elements (12) are radially approached to said gripping axis (X) and a release condition in which the gripping elements (12) are radially distanced from said gripping axis (X), wherein said gripping elements (12) act at a first axial zone of said gripping axis (X) and wherein said gripping elements (12) are configured to grasp and retain a syringe preform (100) when the gripping elements (12) are in the gripping condition;
at least one heating station (23) comprising a heater (24) configured to emit a heating flow directed radially towards said gripping axis (X) at a second axial zone that is distinct and axially separated from said first axial zone of the gripping axis (X);
at least one blow moulding station (25) comprising:
a mould (26) having a portion counter-shaped to a bypass channel (101), wherein the mould (26) is movable between an open condition and a closed condition, wherein in the closed condition the mould (26) is configured to surround the syringe preform (100) and is closed around said gripping axis (X) at a third axial zone of the gripping axis comprising said second axial zone of the gripping axis (X);
a blower (33) configured to deliver pressurized fluid inside said syringe preform (100), wherein said blower (33) is switchable between an active condition in which it delivers a direct pressurized fluid along said gripping axis (X) when the mould (26) is in the closed condition and an inactive condition in which it does not deliver pressurized fluid when the mould (26) is in the open condition.

2. Device (10) according to claim 1, wherein the first axial zone is placed at a flange (103) of said syringe preform (100).

3. Device (10) according to claim 1 or 2, comprising a carousel (22) rotating around a carousel axis (G) and a plurality of said gripping equipment (11) mounted on said carousel (22) radially around said carousel axis (G).

4. Device (10) according to claim 3, comprising a plurality of heating stations (23) placed radially around said carousel axis (G) and not rotatable with said carousel (22).

5. Device (10) according to claim 4, wherein the gripping elements (12) of each gripping equipment are rotatable about respective gripping axes (X) during the rotation of the carousel (22) to expose a same portion of the syringe preform (100) to the heaters (24) of the heating stations (23).

6. Device (10) according to claim 4 or 5, comprising a pick-up station (41) reached by each gripping equipment (11) during the rotation of the carousel (22) around the carousel axis (G); the gripping elements (12) of each gripping equipment (11) moving from the release condition to the gripping condition when they reach said pick-up station (41) in such a way as to pick up a syringe preform (100).

7. Device (10) according to claim 6, wherein said pick-up station (41) comprises a group of actuators (43) configured to insert a syringe preform (100) into the gripping equipment (11) placed at the pick-up station (41).

8. Device (10) according to claim 7, comprising a conveyor (36) that reaches said pick-up station (41); said conveyor (36) comprising a plurality of transport seats (37), configured to transport syringe preforms (100), wherein each transport seat (37) comprises a pair of supports (38) spaced apart from one another along a lying axis (A) and wherein said conveyor (36) comprises guide elements (39) configured to receive in abutment flanges (103) of the syringe preforms (100).

9. Device (10) according to claim 6, comprising an unloading station (51) reached by each gripping equipment (11) during the rotation of the carousel (22) around the carousel axis (G); the gripping elements (12) of each gripping equipment (11) moving from the gripping condition to the release condition when they reach said unloading station (51) in such a way as to release a syringe preform (100).

10. Device (10) according to any one of the preceding claims, comprising a control station (61) configured to identify at least geometric characteristics of the bypass channel (101) formed on the syringe preform (100).

11. Device (10) according to claim 10, comprising a reject station (63) configured to reject a syringe preform (100) whose bypass channel (101) does not have geometric characteristics conforming to a predetermined geometric pattern.

12. Method for making a bypass channel (101) in syringe preforms (100) comprising:
retaining, with a gripping equipment (11), a syringe preform (100) through an end (101a) of the syringe preform (100);
heating with a heating flow a portion of the syringe preform (100) while being retained by the gripping equipment (11);
arranging the syringe preform (100) in a mould (26) having a portion counter-shaped to a bypass channel (101) by orienting the syringe preform (100) with the heated portion at the bypass channel (101);
blowing pressurized fluid inside the syringe preform (100) while it is placed in said mould (26).
